# EUROPEAN PATENT APPLICATION

(11) **EP 4 487 883 A1**
(43) Date of publication of application: **08.01.2025**
(21) Application number: 23183085.2
(22) Date of filing: 03.07.2023
(51) Int. Cl.: A61M 3/02, A61J 1/14, A61M 39/22

(54) **AN IRRIGATION LIQUID CONTAINER**

(71) Applicant: Wellspect AB, 431 21 Mölndal (SE)
(72) Inventor: JOHANSSON, Mathilda, 431 32 Mölndal (SE)
(74) Representative: Lind Edlund Kenamets Intellectual Property AB

(57) **Abstract**

A container for medical use, e.g. for rectal or transanal irrigation, forms a closed compartment. The container comprises a neck extending out from a body of the container and defining an opening at a free end of the neck. An actuator member is connected to the neck and arranged to allow rotation of the actuator member relative to the neck in a circumferential direction of the neck but hinder movement of the actuator member relative to the neck in an axial direction of the neck. A valve member has a closed valve bottom and a valve sleeve, with an opening formed in a side wall of the valve sleeve. The valve sleeve is arranged to be moved in an axial direction upon rotation of the actuator member between a closed position, and an open position. The valve bottom forms a slanted surface.

## Description

### TECHNICAL FIELD OF THE INVENTION

The present invention relates to a container for medical use forming a closed compartment for accommodating a liquid, such as irrigation liquid for use in an irrigation procedure. The container may e.g. be used in rectal or transanal irrigation.

### BACKGROUND OF THE INVENTION

Containers for storing, collecting or transporting liquids are widely used in e.g. the food industry or in the medical field. In the medical field, containers are frequently used for e.g. collecting or storing of body liquids or liquid medications but also for delivery of a liquid to a patient. Such a delivered liquid may be for e.g. intravenous infusion, flushing, transanal irrigation or rectal enema. A liquid to be delivered to a patient for irrigation purpose is often referred to as an irrigation liquid.

Administrating an irrigation liquid is a common medical procedure whereby liquid is injected into a bodily cavity, such as into the rectum and lower intestine of a patient in order to induce bowel movement. The need for such a procedure typically arises in patients suffering from certain physical ailments in which voluntary bowel control is impaired or when the bowel needs to be cleaned before e.g. a coloscopy or a surgical operation. To this end, irrigation systems may be used e.g. by people suffering from spinal cord injuries, spina bifida or multiple sclerosis. For such users, irrigation may improve quality of life by preventing constipation, reducing time spent for bowel emptying procedures, reducing fecal incontinence, and by increasing independency in general.

Such irrigation is nowadays often performed outside medical attendance premises, such as in the patient's home, and is also often performed by the patient himself, i.e. by self-administration. Hereby, the patient needs to do all the required actions, such as filling of the container holding the irrigation liquid, inserting the probe in a correct position, adequately fixating the probe in the bodily cavity, enabling the liquid to be discharged for irrigation and discharge a correct dose of irrigation liquid, and removing the probe after use. Further, many of the users of irrigation systems have reduced dexterity, which makes the operation even more cumbersome.

It is further of importance that the irrigation system is of a limited size, and portable. Portability of the irrigation system is important for disabled persons who are not hospitalized or bed-ridden if they are to live as normal a life as possible. This is particularly important if they travel away from their home, for instance, to someone else's home or if they stay in a hotel. In this situation, they need to be able to deal with their bowel function easily.

Further, for reasons of both cost and sustainability, it is of great importance to reduce waste and to enable reuse of as many parts of the irrigation system as possible.

Several irrigation systems are per se known, such as the ones disclosed in US 10195338, US 10238788, US 9968728, US 9919093 and US 10569008, all by the same applicant. However, there is still a need for making the system more user friendly, in particular for users having poor dexterity, and also more environmentally friendly.

Further, containers useable for such systems are known from US 9610220, US 10350345, also by the same applicant. These containers are collapsible and may be compacted into a compact storage position. However, it has been found that these containers have a limited durability and are only reusable for a very limited number of times. In particular the filling opening is a weak spot, reducing the number of times the container can be reused. The tightness of the seal of the filling opening is rapidly decreasing over time, making the container unfit for use after only 10-15 times of use. This problem is particularly present in case the system uses indirect pumping of the irrigation liquid. Indirect pumping of the irrigation liquid has been found to be of great advantage in irrigation procedures in general, and in particular for rectal and transanal irrigation. By indirect pumping is here meant that air is pumped into the container to increase the pressure therein, thereby forcing liquid in the container to be pumped out.

Thus, there is a need for a container for medical devices, and in particular irrigation devices, which can be reused over more cycles, and thereby make the container and the entire irrigation system more user friendly and more sustainable. There is also a need for a container which can be handled more easily, in particular for users having poor dexterity.

Thus, there is a need for an improved container for medical use, such as for holding and delivering of an irrigation liquid.

### SUMMARY OF THE INVENTION

It is therefore an object to provide a container for medical use which at least alleviates the above-discussed drawbacks of the prior art. In particular, it is an object to provide a container for accommodation of irrigation liquid for use in irrigation procedures, such as in rectal or transanal irrigation.

According to a first aspect of the present disclosure there is provided a container for medical use forming a closed compartment for accommodating a liquid, comprising:
a neck extending out from a body of the container and defining an opening at a free end of the neck;
an actuator member connected to the neck and arranged to allow rotation of the actuator member relative to the neck in a circumferential direction of the neck but hinder movement of the actuator member relative to the neck in an axial direction of the neck; and
a valve member having a closed valve bottom and a valve sleeve, wherein the valve bottom is connected to the valve sleeve, with an opening formed in a side wall of the valve sleeve in the vicinity of the valve bottom, wherein the valve sleeve is connected to the actuator member, and arranged to interact with the actuator member to be moved in an axial direction relative to the neck and the actuator member upon rotation of the actuator member;
wherein the valve member is axially displaceable between a closed position, in which the valve bottom is arranged relatively closer to the actuator device and a seal is formed between the valve bottom and the neck, and an open position, in which the valve bottom is arranged relatively farther from the actuator device, and wherein the valve bottom forms a slanted surface. The slanted surface is preferably directed or inclined towards the neck opening, whereby the slanted surface is inclined and non-parallel to both the axial and radial direction of the neck.

The closure provided by the actuator member and the valve member is very efficient and useful.

The closure can be made very easy to open and close, which is of great advantage for users having poor dexterity, low hand and finger strength, etc. It also makes it possible to open and close the container opening using only one hand, or by use of other body parts, such as an elbow or the like. Since the closure is operated by twisting and rotating the actuator member, which is easily accessible, the actuator member could be provided with gripping means, etc., which facilitates the opening and closing procedure even further. For example, the actuator member may be provided with one or more radially protruding gripping handles, which facilitates gripping and functions as lever arms when operating the actuator member.

The rotational movement of the actuator member is translated into an axial movement for the valve member. Depending on e.g. the pitch of a threaded connection between the valve member and the actuator member, the length of the axial translation resulting from a given rotational movement could be controlled and chosen. It has been found that a relatively small rotational movement could be used to obtain a sufficient axial movement, such as less than 90 degrees, and preferably less than 60 degrees, and more preferably less than 50 degrees, such as about 45 degrees. Small rotational movements are generally easier to perform for users having poor dexterity, as well as for users in general.

The valve member is preferably connected to the neck in such a way that rotational movement of the valve member relative to the neck is prevented, or at least limited. Hereby, the valve sleeve is not, or only to a limited extent, rotated by the rotational movement of the actuator member. Thus, the actuator member is moveable in a rotational direction relative to the neck, but not moveable, or moveable only to a limited extent, in an axial direction. Similarly, the valve member is moveable in an axial direction relative to the neck, but not moveable, or moveable only to a limited extent, in a rotational direction. To restrict rotational movement for the valve member in relation to the neck, a guide path and a protruding guide element may e.g. be used on adjacent surfaces formed on the valve member and the neck. For example, the neck may be provided with one or more guide paths in the form of a guide groove, extending essentially in the longitudinal direction, and the valve member may be provided with one or more guide elements, such as a protruding knob or pin, arranged to be guided in the guide path.

Further, the herein disclosed closure provides a very efficient sealed closure, thereby alleviating the risk of spillage and leakage, and also enabling the container to be pressurized, e.g. for indirect pumping of an irrigation liquid, where air is pumped into the container and a liquid accommodated in the container is pumped out due to the increased pressure. Such indirect pumping is commonly used in rectal and transanal irrigation systems. The disclosed closure is efficient for maintaining the appropriate pressure in the container for a long period of time. Since closing of the valve member occurs in a direction from the inside of the container to the outside, an increased pressure in the container in fact increases the tightness of the seal, since the increased pressure aids in pressing the valve member, and in particular the valve bottom, towards the neck.

The disclosed closure also involves relatively few and simple components, thereby making both production and assembling of the container time and cost-efficient.

In addition, the container, and in particular the closure, is very durable, thereby making it possible to repeatedly open and close the closure, enabling an efficient reuse of the container.

For a container in an irrigation system, and in particular a rectal or transanal irrigation system, the neck opening may be used for filling the container with an irrigation liquid, such as water, prior to every irrigation procedure, and possibly for releasing any residual liquid remaining after completion of the irrigation procedure. Thus, a typical irrigation procedure may involve at least two cycles of opening and closing the closure. The presently disclosed closure could be opened and closed many times without degradation, and with maintained full functionality. This enables reuse of the container for several weeks or months, even if irrigation is made frequently, such as every day. It has been found that the present closure could have a duration of at least 100 cycles without any damage or deterioration.

The slanted surface of the valve bottom, in particular directed towards the neck opening, provides a smoother path for a liquid, such as water, being filled into the container. Hereby, backflow, turbulence, etc., at, or in the vicinity of, the opening in the sidewall of the valve sleeve is avoided. Such turbulence and backflow may otherwise cause a risk of overflow and a reduced inflow rate. Due to the slanted valve bottom filling can be made quicker. Further, the risk of spilling, splashing etc. is also efficiently reduced.

A further advantage of the presently disclosed container is that nothing from the closure is removed upon opening, or made to protrude from the container. Instead, the outer appearance of the closure and the container will remain essentially the same regardless of whether the valve member is in the opened or closed position, and nothing is ever removed from the container. Hereby, the risk of inadvertently misplacing or damaging any part of the container and closure is efficiently reduced.

The opening in the side wall of the valve sleeve in the vicinity of the valve bottom may be formed around essentially the whole circumference of the valve member. However, alternatively, the opening may have a limited extension, extending over only part of the circumference. Preferably, the opening has an angular extension of at least 90 degrees, and preferably of at least 180 degrees. Further, the opening is preferably evenly distributed over the circumference.

The opening in the side wall of the valve sleeve is preferably arranged directly abutting the valve bottom.

The neck may comprise a first engagement structure extending circumferentially in a closed circular path around an external surface of the neck, and the actuator member may comprise a second engagement structure extending circumferentially in a closed circular path around an internal surface, the first and second engagement structures being arranged to allow rotation of the actuator member relative to the neck in a circumferential direction of the neck but hinder movement of the actuator member relative to the neck in an axial direction of the neck.

In the context of the present invention, to hinder movement of the actuator member relative to the neck in an axial direction of the neck generally means that such axial movement is prohibited or stopped, or that only a very limited axial movement is possible, due to e.g. manufacturing tolerances.

One of the first and second engagement structure may comprise a groove, extending in a circle around the surface, and the other engagement structure may comprise a protrusion. The protrusion may comprise a continuous protruding line. However, the protrusion may also comprise sections of protruding lines arranged along a circle. The sections may have various lengths, and may e.g. be in the form of pins etc.

The first and second engagement structure may be arranged to be snap connected to each other.

The actuator member may comprise an outer tube portion and an inner tube portion, the outer and inner tube portions being connected by an annular surface extending between a top end of the outer tube portion and a top end of the inner tube portion, wherein the actuator member is arranged to receive the neck between the outer tube portion and the inner tube portion.

The second engagement structure may extend circumferentially in a closed circular path around an internal surface of the outer tube portion.

The valve sleeve may be arranged positioned between the neck and the inner tube portion of the actuator member. However, alternatively, the valve member may be arranged inside the neck and the actuator member. In such embodiments, the actuator member does not need to include any inner tube portion.

According to another aspect of the present disclosure, there is provided a container for medical use forming a closed compartment for accommodating a liquid, comprising:
a neck extending out from a body of the container and defining an opening at a free end of the neck and a first engagement structure extending circumferentially in a closed circular path around an external surface of the neck;
an actuator member having an outer tube portion and an inner tube portion, the outer and inner tube portions being connected by an annular surface extending between a top end of the outer tube portion and a top end of the inner tube portion, wherein the actuator member is arranged to receive the neck between the outer tube portion and the inner tube portion and wherein the outer tube portion has a second engagement structure extending circumferentially in a closed circular path around an internal surface of the outer tube portion, the first and second engagement structures being arranged to allow rotation of the actuator member relative to the neck in a circumferential direction of the neck but hinder or prohibit movement of the actuator member relative to the neck in an axial direction of the neck; and
a valve member having a closed valve bottom and a valve sleeve, wherein the valve bottom is connected to the valve sleeve, with an opening formed in a side wall of the valve sleeve in the vicinity of the valve bottom, wherein the valve sleeve is arranged to be positioned between the neck and the inner tube portion of the actuator member, and arranged to interact with the actuator member to be moved in an axial direction relative to the neck and the actuator member upon rotation of the actuator member;
wherein the valve member is axially displaceable between a closed position, in which the valve bottom is arranged relatively closer to the actuator device and a seal is formed between the valve bottom and the neck, and an open position, in which the valve bottom is arranged relatively farther from the actuator device, and wherein the valve bottom forms a slanted surface.

The slanted surface of the valve bottom is preferably rotationally symmetric around a longitudinal axis of the valve member and with the part of the surface being closest to the longitudinal axis being closest to the actuator member. Thus, the central part of the valve bottom may form the highest point of the surface, or alternatively be part of an area of the same height, forming a raised plateau.

The slanted surface of the bottom may be realized in various ways, such as by a generally straight slanted surface, or various shaped, curved surfaces, such as a surface having a conical shape. In a preferred embodiment, the slanted surface of the valve bottom forms a conical surface. The conical surface may be in the form of a straight cone, but may alternatively be in the form of a convex or concave cone. In a preferred embodiment, the slanted surface forms a concave cone. A concave cone has a steeper, higher inclination at the center, a less steep, lower inclination at the peripheral edge, and a gradual transition between the high inclination and the low inclination between the center and the periphery.

The seal is preferably formed between an outer surface of the valve bottom and an inner surface of the neck, both said outer surface and inner surface being orthogonal to the axial direction of the neck and valve member. Hereby, the seal is formed as a cylindrically shaped interface. In such an embodiment, the seal properties will always be the same, regardless of the force applied to the actuator member, and there is no risk of damaging the seal forming parts by overtightening, etc. The seal will also remain the same even after many use cycles, and is hereby very durable and reliable. Still further, very low force is needed to form the seal when the valve member is moved to the closed position. Thus, this type of seal is also highly advantageous for users having poor dexterity, low hand strength, etc.

However, the seal may also be arranged between other surfaces, such as an upwardly directed surface of the valve member and a downwardly directed surface of the neck.

The container may further comprise a gasket, arranged on at least one of the valve bottom and the neck, wherein the gasket is arranged to be positioned between the valve bottom and the neck when the valve member is in the closed position. The gasket ensures that a good, tight seal is obtained when the valve member is brought to the closed position. The gasket may e.g. be an O-ring. The gasket is preferably arranged on, or integrated with, the valve member. However, the gasket may alternatively be arranged on, or integrated with, the neck. It is also feasible to use two or more gaskets. For example, a gasket may be arranged on, or integrated with, both the neck and the valve member.

The gasket may be of a material which less rigid and more flexible than the other parts of the container. The gasket may e.g. be made of rubber, a thermoplastic elastomer (TPE), etc.

The gasket may be formed as a separate part, and connected to the valve member and/or the neck by adhesive or the like. Alternatively, or additionally, the gasket may be held in place mechanically, such as being arranged in a groove or the like. For example, the valve member may have a groove facing radially in an outward direction.

Alternatively, the gasket may be formed as an integrated part of the valve member or the neck, such as being formed by a less rigid material in a 2K injection molding process.

In a preferred embodiment, the gasket is arranged on the valve bottom and arranged to protrude radially out past a perimeter of the valve bottom.

The actuator member may be arranged to facilitate gripping and maneuvering. For example, the actuator member may be provided with a corrugated outer surface, have a friction increasing pattern, or in other ways having a structure increasing the friction. In a preferred embodiment, the actuator member comprises one or more radially projecting handles. For example, two such radially projecting handles may be provided, preferably extending in opposite directions. In such an embodiment, the actuator member may have a shape, seen from above, resembling an oval, and possibly with pointed ends, such as an almond shape. However, many other shapes are also feasible, such as rectangular, triangular, star shaped, etc.

The valve member is preferably arranged to move between the closed position and the open position upon less than 180 degrees rotation of the actuator member, and preferably less than 90 degrees, and most preferably less than 60 degrees. A relatively small angular rotation is of advantage since it enables the user to perform the entire rotation without changing grip, without changing arm and body position, etc. On the other hand, a too small rotational angle would make the force required to obtain the rotation relatively high. The valve member is preferably arranged to move between the closed position and the open position upon more than 20 degrees rotation of the actuator member, and preferably more than 30 degrees, and most preferably more than 40 degrees. For example, the actuator member can be rotated about 45 degrees for opening and closing the valve member.

The container preferably comprises a side wall member formed by a flexible material having a first and second open end, a rigid bottom portion sealingly arranged at said first open end of said side wall member and a rigid top portion sealingly arranged at said second open end of said side wall member, wherein the rigid top portion includes said neck. Preferably, the side wall member is flexible such that said container is reversibly foldable and unfoldable, thereby being arrangeable in a compact state, in which the rigid top and bottom portions are arranged relatively closer to each other, and in an expanded state, in which the rigid top and bottom portions are arranged relatively further apart, respectively. In particular, the containers may be realized in the way disclosed in US 9610220 and US 10350345 by the same applicant, said documents hereby being incorporated by reference in their entirety. The closure arrangement discussed in the foregoing may in such an embodiment be arranged in the rigid top portion.

The closure arrangement is preferably used as a filling opening, for filling the container with an irrigation liquid prior to use.

An outlet opening for pumping liquid out from the container may be arranged in the vicinity of the filling opening, such as in the rigid top portion. However, in other embodiments, the opening in the neck forms a filling opening for the container and a second opening is provided in the rigid bottom portion, said second opening forming a discharge opening.

The actuator member is preferably operatively connected to the valve member by a threaded connection, formed between an outer or inner surface of the inner tube portion of the actuator member and an inner or outer surface of the valve sleeve of the valve member. Alternatively, the threaded connection may be formed between a surface of the outer tube portion of the actuator member and an outer surface of the valve sleeve. The threaded connection may comprise a helical groove, formed in one of the outer surfaces of the inner tube portion and the inner surface of the valve sleeve. A complementary protruding element is formed on the other surface. The complementary protruding element may be in the form of a helical thread. However, alternatively, the protruding element may be in the form of one or more protruding pins or sections.

The container is preferably adapted for use in an irrigation system, and preferably a rectal or transanal irrigation system. In particular, the container is useable for indirect pumping, where air is pumped into the container for increasing the pressure in the container, thereby to pump, indirectly, liquid out from the container.

According to a further aspect of the present disclosure, an irrigation system, and preferably a rectal or transanal irrigation system, is provided, comprising a container as discussed in the foregoing.

These and other aspects of the invention will be apparent from and elucidated with reference to the embodiments described hereinafter.

### BRIEF DESCRIPTION OF THE DRAWINGS

For exemplifying purposes, the invention will be described in closer detail in the following with reference to embodiments thereof illustrated in the attached drawings, wherein:
Fig. 1 illustrates an irrigation system in accordance with an embodiment of the present disclosure;
Fig. 2 schematically illustrates a side view of a container in accordance with an embodiment of the present disclosure;
Fig. 3 schematically illustrates a side view of a container in accordance with another embodiment of the present disclosure;
Figs. 4-6 and 7a-c show a closure arrangement of a container in accordance with the present disclosure, wherein Fig. 4 is an exploded perspective view of the components forming the closure arrangement, Fig. 5 is a cross-sectional sideview of the closure arrangement in a closed position, Fig. 6 is a perspective view of the valve member, and Figs. 7a-c are cross-sectional views, partly in perspective, illustrating the valve arrangement in a closed, partly opened and opened state, respectively;
Figs. 8a-c are schematic illustrations of the closure arrangement illustrating various possible positions for the gasket with a radial closing interface;
Figs. 9a-c are schematic cross-sectional illustrations of the closure arrangement illustrating various possible positions for the gasket with an axial closing interface;
Figs. 10a-d are schematic cross-sectional illustrations of various slanted surface shapes of the valve bottom;
Figs. 11a-f are schematic top view illustrations of various embodiments of the actuator member; and
Fig. 12 is a schematic cross-sectional view of one side of a closure arrangement in accordance with another embodiment.

### DETAILED DESCRIPTION OF CURRENTLY PREFERRED EMBODIMENTS

The container of the present disclosure is particularly useful for irrigation systems, and in particular rectal and transanal irrigation systems. However, the container may also be used in other types of medical devices, and in particular medical devices where there is a need to fill or refill the container with a liquid prior to use. The following discussion is, in particular, concerned with the preferred field of use, rectal and transanal irrigation systems, even though the disclosure is not limited to this particular type of containers.

It is to be understood that features of the different embodiments are exchangeable between the embodiments and may be combined in different ways, unless anything else is specifically indicated. It may also be noted that, for the sake of clarity, the dimensions of certain components illustrated in the drawings may differ from the corresponding dimensions in real-life implementations of the invention, e.g. the length and width of the container, etc.

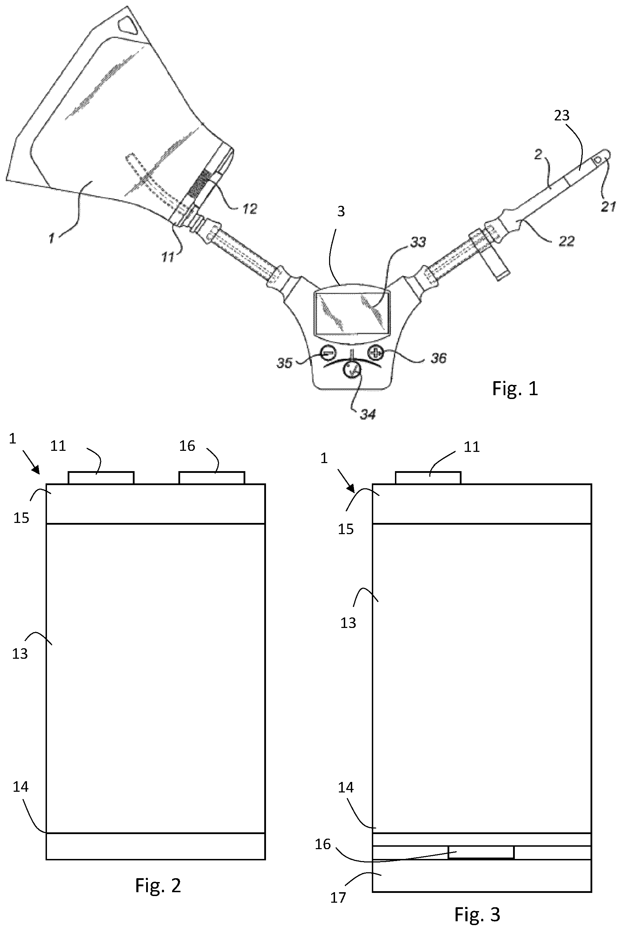

Fig. 1 discloses an irrigation system according to a first exemplary embodiment, comprising a container 1 for an irrigating liquid, a probe 2 for arrangement in a user, and a control unit 3.

The container may be realized in various ways, as will be discussed in more detail in the following. For example, the container may be formed by a rigid, semi-rigid or flexible material. In case a semi-rigid or flexible material is used, the container may be collapsible or foldable, to make the irrigation system more compact prior to use. The container is provided with an opening, closed by a closure 11, for filling of the reservoir. Tubing connecting the container to the rest of the irrigation system may be provided through the closure 11, or through other access points on the container.

In order to render the irrigation system as portable as possible, the container preferably has a capacity of less than 5 liters, more preferred less than 3 liters and most preferred less than 2 liters. If however the system is to be used for repeated irrigation, a larger capacity container may be necessary.

The container may comprise an overpressure release valve, to release pressure over a predetermined maximum pressure to be allowed. Further, the container may also, optionally, comprise a filter 12, such as a hydrophobic filter, which is impermeable to the irrigation liquid, but which allows air to enter the reservoir but not escape the container. Such a filter ensures that the container maintains its shape when irrigation liquid is being pumped out from the container.

A pump (not shown) may be used to pump air into the container for, indirectly, pumping liquid out of the container. Alternatively, the pump may directly pump liquid out of the container.

The probe 2 is provided with a retention member, such as an inflatable balloon 23, in the vicinity of an insertable end 21, for fixing the catheter in a body cavity. Further, the probe may be provided with a rearward enlarged part 22, providing an abutment to hinder too deep insertion. The probe is provided with two lumens - one lumen for transfer of irrigation liquid through the probe, for discharge at the forward end, and one lumen for inflation and deflation of the balloon.

The pump may be a manually operated pump, such as a bulb pump, or an electric pump.

A control unit may be provided. The control unit may be realized as a unitary, handheld unit. The control unit may be arranged connected to the container and the probe. Alternatively, the container may be directly connected to the probe. In such realizations, the control unit may e.g. be arranged as a remote control, controlling a pump connected to the container. The control unit may comprise a display 33, and control elements 34, 35 and 36. The control elements may be realized as a depressible control button.

The irrigation liquid can be any liquid which is capable of irrigating the body cavity of interest. In order to stimulate bowel movements, suitable irrigation liquids include water, hypertonic aqueous salt solutions, solutions or suspensions of cathartic agents, such as bisacodyl or phenolphthalein, and mineral oil.

Referring to Figs. 2 and 3, the container 1 may comprise a flexible side wall member 13, a rigid bottom portion 14, a rigid top portion 15, an inlet opening 11, and an outlet opening 16.

In the embodiments illustrated in Figs. 2 and 3, when assembled, the bottom portion 14 is arranged at a first open end of the side wall member 13 such that it seals the first open end. The rigid top portion 15 is arranged at a second open end of the side wall member 13 such that it seals the second open end.

The term "top" and "bottom" here refers to one possible way of orienting the container during use, or during part of the use, such as during filling of the container. However, it should be appreciated by the skilled artisan that the container may also be used in other orientations, such as with the bottom portion arranged above the top portion.

In the embodiment of Fig. 2, both a filling opening 11 and an outlet opening 16 are provided in the rigid top portion. However, alternatively, as illustrated in Fig. 3, the outlet opening may instead be provided in the rigid bottom portion 14, which may e.g. be docked into a base portion 17.

In particular, the filling opening 11 will be discussed in more detail in the following. However, this closure arrangement may also be used for the outlet opening 16 or for other openings in the container.

The side wall 13 may be made from a flexible sheet material. The material may be a plastic material such as polypropylene, polyimide, polyethylene, EBA, or combinations thereof. The side wall member 13 may be cylindrical in the expanded state, but rectangular shapes are also feasible, such as hexagonal shapes and other shapes.

The term "rigid" here indicates that the top and bottom portions are more rigid, and preferably substantially more rigid, than the side wall member. Preferably, the top and bottom portions maintain their shape during normal use.

By arranging a collapsible wall between relatively rigid bottom and top portions, a very stable container is obtained, which may still be highly compressible. A compressible container is easy to fill because the size of the container is relatively compact, with an efficient space utilization, and the shape may also easily be adapted to e.g. different sized sinks. The container is also easy to fill in situations where a tap or filling station is at a different angle. In such case, the container may be appropriately flexed at the compressible side walls.

The container may be very compact in the compacted disposition. Due to this compacted disposition, storing and transporting of the container, or a plurality of containers may be more efficient. In particular, the container may, in the compacted state, be relatively flat and disk shaped, which makes the container easy to carry around, e.g. in a pocket or a bag.

The rigid top and bottom portions make the container have a high stability while having flexible side walls. This is advantageous because it may allow e.g. a stable up-right position of the container when in use.

According to an embodiment of the present disclosure, in the compact state, the top and bottoms portions are in contact with each other. This way, the size of the container is efficiently reduced for e.g. storage, filling, or transportation. Contact may be made at one or several contact points. At contact the top and bottom portions may be essentially parallel to each other. Hereby, the height of the container in the compact state will essentially correspond to the height of the top and bottom portions.

The top and bottom portions are preferably essentially equal in diameter and circumferential shape. This allows the side wall member to be shaped as a tube with even diameter. However, the top and bottom portions may also have different dimensions and/or shapes. Hereby, one of the top and bottom portions may e.g. be allowed to be at least partly accommodated by the other in the collapsed state.

Furthermore, in the compact state, the top and bottoms portions may be rotated relative each other compared to when in the expanded state. This is advantageous because it may allow the size of the container to be further reduced when in the compacted state. In the compacted disposition the flexible side walls may collapse in a direction essentially parallel to a longitudinal direction of the container The side walls may collapse in both the longitudinal direction and in a transverse direction. However, collapsing solely in the longitudinal direction is also possible.

In the compact state, the container height is preferably shorter than the height in the expanded state, and preferably less than 1/2, and more preferably less than 1/4. This is advantageous because it allows more size efficient storage or transportation of the container. It may further allow filling of the container from taps or filling stations with reduced or cramped space. Preferably, the height of the container in the collapsed state is less than 5 cm, and most preferably less than 3 cm for a container capable of holding at least 1 liter in the expanded state.

The flexible side wall may be formed in various ways. It may e.g. be produced as a tubular member by means of extrusion or injection molding. It may also be formed by one or several sheet materials, e.g. connected by means of welding or adhesion. However, other production methods may also be used, such as vacuum forming and the like.

A closure arrangement will now be discussed in greater detail. The closure arrangement is particularly suitable for the inlet opening, i.e. the filling opening, of the above-discussed container, but may also be used for the outlet opening and for inlet and outlet openings of other types of medical use containers.

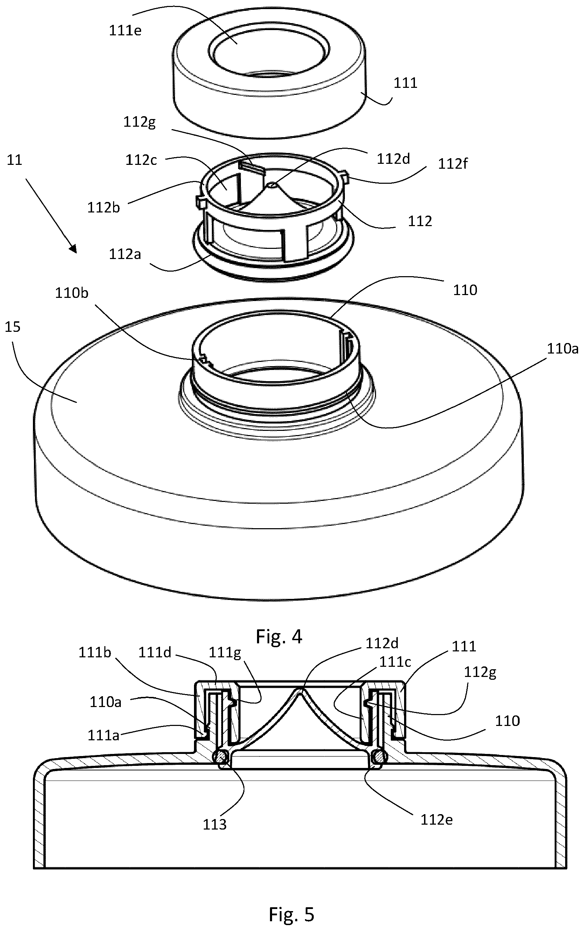

In an embodiment, as shown in Figs. 4-6 and 7a-c, the closure arrangement at the opening 11 comprises a neck 110 extending out from a body of the container, such as being arranged on and extending out from the rigid top portion 15, and defining an opening at a free end of the neck.

Further, the closure arrangement may comprise an actuator member 111 connected to the neck and arranged to allow rotation of the actuator member relative to the neck 110 in a circumferential direction of the neck but hinder or prohibit movement of the actuator member relative to the neck in an axial direction of the neck. The closure arrangement may further include a valve member 112 having a closed valve bottom 112a and a valve sleeve 112b, wherein the valve bottom 112a is connected to the valve sleeve 112b, with an opening 112c formed in a side wall of the valve sleeve in the vicinity of the valve bottom, and preferably abutting the valve bottom 112a. The valve sleeve 112b is preferably connected to the actuator member 111, and arranged to interact with the actuator member to be moved in an axial direction relative to the neck 110 and the actuator member 111 upon rotation of the actuator member.

Due to the axial movement of the valve member 112 it is axially displaceable between a closed position, in which the valve bottom 112 is arranged relatively closer to the actuator device 111 and a seal is formed between the valve bottom 112a and the neck 110, and an open position, in which the valve bottom 112a is arranged relatively farther from the actuator device 111.

The valve member is preferably connected to the neck in such a way that rotational movement of the valve member relative to the neck is prevented, or at least limited. Hereby, the valve sleeve is not, or only to a limited extent, rotated by the rotational movement of the actuator member. To restrict rotational movement for the valve member in relation to the neck, a guide path and a protruding guide element may e.g. be used on adjacent surfaces formed on the valve member and the neck. For example, the neck may be provided with one or more guide paths in the form of a guide groove 1 10b, extending essentially in the longitudinal direction, and the valve member may be provided with one or more guide elements, such as a protruding knob or pin 112f, arrange to be guided in the guide path.

The valve bottom 112a preferably forms a slanted surface 112d directed or inclined towards the neck opening. The slanted surface of the valve bottom directed or inclined towards the neck opening provides a smoother path for a liquid, such as water, being filled into the container.

Thus, the rotational movement of the actuator member 111 is translated into an axial movement for the valve member 112. Depending on e.g. the pitch of a threaded connection between the valve member and the actuator member, the length of the axial translation resulting from a given rotational movement could be controlled and chosen. It has been found that a relatively small rotational movement, such as about 45 degrees, could be used to obtain a sufficient axial movement.

In the illustrative embodiment, the neck 110 comprises a first engagement structure 110a extending circumferentially in a closed circular path around an external surface of the neck. The first engagement structure may be in the form of an outwardly protruding annular ridge, as in the illustrated embodiment, but may also be in the form of an annular groove or the like. The actuator member 111 may comprise a second engagement structure 111a extending circumferentially in a closed circular path around an internal surface. In the illustrative example, the second engagement structure 111a is in the form of an inwardly protruding annular ridge, such as an annular flange. The first and second engagement structures may form a snap lock connection to each other, and are arranged to allow rotation of the actuator member relative to the neck in a circumferential direction of the neck but hinder or prohibit movement of the actuator member relative to the neck in an axial direction of the neck.

In the above-discussed embodiment, both the first and second engagement structure comprises an annular protrusion. However, alternatively one of the first and second engagement structure may comprise a groove, extending in a circle around the surface, and the other engagement structure may comprise a protrusion.

Further, the annular protrusions may comprise a continuous protruding line. However, at least one of the annular protrusions may also comprise sections of protruding lines or point like protrusions arranged along a circle. The sections may have various lengths, and may e.g. be in the form of pins etc.

The actuator member 111 may comprise an outer tube portion 111b and an inner tube portion 111c, the outer and inner tube portions being connected by an annular surface 111d extending between a top end of the outer tube portion 111b and a top end of the inner tube portion 111c, wherein the actuator member is arranged to receive the neck 110 between the outer tube portion 111b and the inner tube portion 111c.

The outer tube portion 111b, the inner tube portion 111c and the annular surface 111d collectively define a U-shaped channel at the top end of the actuator member 111. The U-shaped channel receives the neck 110 and the valve sleeve 112b. The U-shaped channel may also form a stop for the valve member 112, preventing axial movement for the valve member in an upward direction when the inner surface of the channel has been reached.

The outer tube portion 111b generally includes a tubular body having an inner diameter approximately the same as, or only slightly larger than, the outer diameter of the neck 110.

The second engagement structure 111a may extend circumferentially in a closed circular path around an internal surface of the outer tube portion 111b.

The valve sleeve 112b may be arranged positioned between the neck 110 and the inner tube portion 111c of the actuator member 111.

The valve sleeve 112b preferably has an outer diameter approximately the same as, or only slightly smaller than, an inner diameter of the neck 110. Further, the valve sleeve 112b preferably has an inner diameter approximately the same as, or only slightly larger than, the outer diameter of the inner tube portion 111c of the actuator member 111.

A through opening 111e is formed centrally within the inner tube portion 111c, defining a fluid passage through which a liquid may flow into the container when the opening is used as a filling opening, or out from the container when the opening is used as an outlet opening. The valve member 112 is operable relative to the fluid passage defined in the actuator member 111 and the neck 110 to form the opened and closed positions. The actuator member 111 is rotatably supported by the neck 110 and operatively connected to the valve member 112 for opening and closing the valve member relative to the fluid passage.

The valve member 112 is made to move axially upon rotation of the actuation member 111, whereby the rotational movement is translated into an axial movement.

To this end, the actuator member 111 is preferably operatively connected to the valve member 112 by a threaded connection, formed between a surface of the inner tube portion 111c of the actuator member and a surface of the valve sleeve 112b of the valve member 112 facing said surface of the inner tube portion 111c. The threaded connection may comprise a helical groove 111g, formed in one of the surfaces of the inner tube portion and the surface of the valve sleeve. A complementary protruding element is formed on the other surface. The complementary protruding element 112g may be in the form of a helical rib, forming a thread. However, alternatively, the protruding element may be in the form of one or more protruding pins, or as a section of a helical path, as in the illustrative example. In the shown example, a helical groove 111g is formed on the surface of the inner tube portion 111c directed towards the neck, whereas two radially outward protruding helical section 112g are provided on the valve member 112, on a surface directed away from the neck. It may also suffice with only one pin or helical section, or alternatively more than two pins/sections may be used. The pins/sections are preferably arranged evenly distributed around the circumference. However, it is likewise possible to arrange the thread on the valve member and the protrusion or helical section on the actuator member.

The protruding element(s)/section(s) 112g and the helical groove together form a mating threaded connection. Due to the threaded connection formed between the actuator member and the valve member, rotating the actuator member while the actuator member remains fixed in place in the axial direction relative to the neck causes the valve member to be displaced in the axial direction between the open and closed positions thereof depending upon the direction of rotation of the actuator member. Due to the guiding path 110b and the protruding guide elements 112f, the valve member is not rotated by the actuator member, or with only very limited rotation.

The valve bottom 112a forms a closed surface extending between the peripheral circumferences. The closed surface of the valve bottom forms the slanted surface 112d in an upward direction. The slanted surface is preferably rotationally symmetric around a longitudinal axis of the valve member and with the part of the surface being closest to the longitudinal axis being closest to the actuator member. Thus, the central part of the valve bottom may form the highest point of the surface, or alternatively be part of an area of the same height, forming a raised plateau.

In a preferred embodiment, the slanted surface 112d of the valve bottom forms a conical surface. The conical surface may be in the form of a straight cone, but may alternatively be in the form of a convex or concave cone. In the illustrative embodiment, the slanted surface 112d forms a concave cone. A concave cone has a steeper, higher inclination at the center, a less steep, lower inclination at the peripheral edge, and a gradual transition between the high inclination and the low inclination between the center and the periphery.

The opening 112c in the side wall of the valve sleeve in the vicinity of the valve bottom may be formed around essentially the whole circumference of the valve member 112, as in the illustrative example.

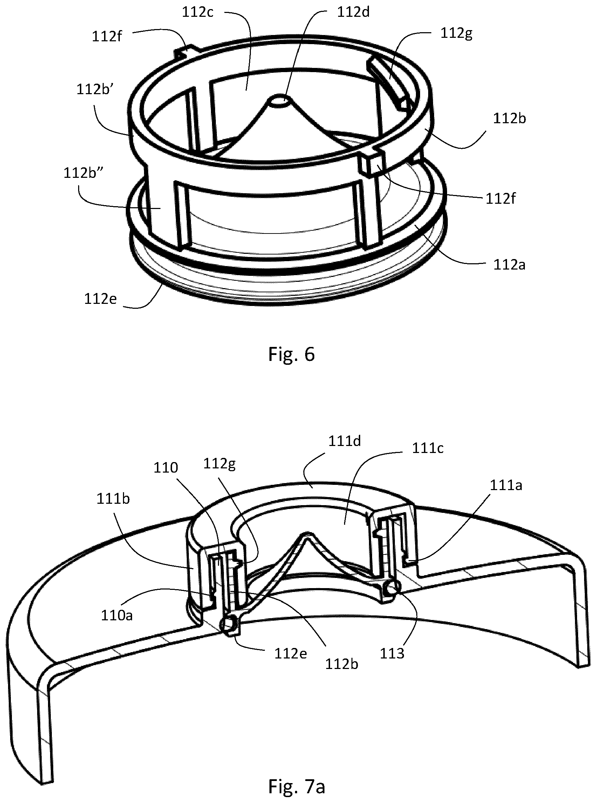

In the illustrative example, best seen in Fig. 6, the valve sleeve 112b may comprise a closed, annular structure 112b', extending around the circumference of the valve sleeve, and arms or pillars 112b" connecting the closed, annular structure 112b' to the valve bottom 112a, and forming the opening 112c therebetween. In the illustrative example four arms 112b" are provided, evenly distributed around the circumference, forming four separate parts of the opening 112c.

However, other realizations are also feasible, such as having only two or three arms, or more than four arms, such as five or six arms. The arms need also not have constant widths, but may instead have a triangular shape, an hourglass shape, or the like.

In the illustrative example, the parts of the opening 112c are evenly distributed around the circumference, and apart from the thin arms 112b' the openings extend essentially over 360 degrees of the circumference. However, alternatively, the opening may have a limited extension, extending over only part of the circumference. Preferably, the opening has an angular extension of at least 90 degrees, and preferably of at least 180 degrees. Further, the opening is preferably evenly distributed over the circumference. However, alternatively, the opening may be primarily, or solely, provided in one or more sectors only.

The opening 112c in the side wall of the valve sleeve is preferably arranged directly abutting the valve bottom 112a, as in the illustrative example. However, it is also feasible to arrange the opening at a slight distance from the valve bottom 112a.

In this embodiment, the seal is preferably formed between an outer surface of the valve bottom and an inner surface of the neck. Both these surfaces are orthogonal to the axial direction of the neck and valve member. Hereby, the seal is formed as a cylindrically shaped interface.

In an embodiment, an annular gasket 113, such as an O-ring, is arranged on the outer surface of the valve bottom. The O-ring may e.g. be arranged in an annular groove 112e formed in the outer surface of the valve bottom 112a and arranged to face in a radial direction towards the inner wall of the neck 110. The dimensions of the annular groove 112e and the gasket 113 are such that the gasket extends out from the valve bottom and the annular groove in a radial direction. Hereby, the gasket 113 is positioned between the valve bottom 112a and the neck 110, and bears against the inner surface of the neck 110, when the valve member is in the closed position. The gasket ensures that a good, tight seal is obtained when the valve member is brought to the closed position.

The gasket may be of a material which less rigid and more flexible than the other parts of the container, and in particular less rigid and more flexible than the neck and the actuator member. The gasket may e.g. be made of rubber, a thermoplastic elastomer (TPE), etc.

The gasket may be formed as a separate part and connected to the valve member and/or the neck by adhesive or the like. Alternatively, or additionally, the gasket may be held in place mechanically, such as being arranged in a groove or the like, as in the illustrative example.

Alternatively, the gasket may be formed as an integrated part of the valve member or the neck, such as being formed by a less rigid material in a 2K injection molding process.

The actuator member 111 may be arranged to facilitate gripping and maneuvering. For example, the actuator member may be provided with a corrugated outer surface, have a friction increasing pattern, or in other ways having a structure increasing the friction.

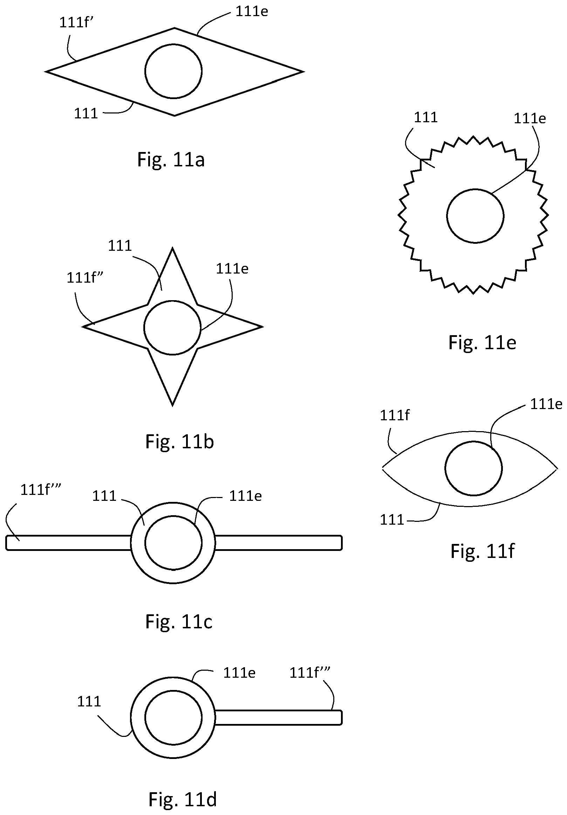

The actuator member may have a generally circular cross-sectional shape. However, in a preferred embodiment, the actuator member comprises one or more radially projecting handles 111f (see Figs. 11a-f). For example, two such radially projecting handles 111f may be provided, preferably extending in opposite directions. In such an embodiment, the actuator member may have a shape, seen from above, resembling an oval, and possibly with pointed ends, such as an almond shape, as in the illustrative example. However, many other shapes are also feasible, such as rectangular, triangular, star shaped, etc. The radially protruding handles 111f provide a lever to facilitate rotation of the actuation member 111.

The valve member is preferably arranged to move between the closed position and the open position by a rotation in the range of 20-180 degrees of the actuator member, and preferably in the range of 30-90 degrees, and most preferably in the range of 40-60 degrees, such as about 45 degrees.

Operation of the closure arrangement is illustrated in Figs. 7a-7c. In Fig. 7a, the valve member 112 is in its uppermost position, in the same way as illustrated in Fig. 5, wherein a seal is formed between neck 110 and the valve bottom 112a, and the possible gasket 113 arranged on the outer side of the valve bottom. This sealed connection together with the closed valve bottom closes the opening formed through the neck.

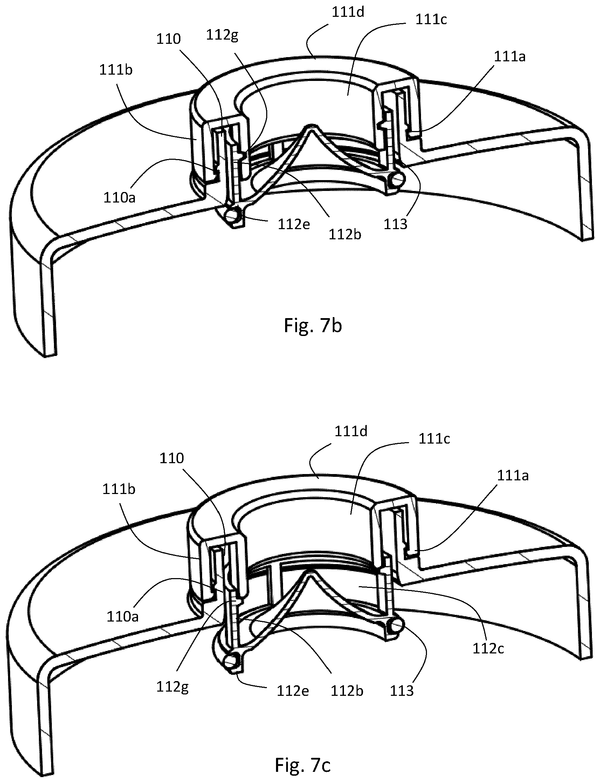

In fig. 7b, the actuator member 111 has been slightly rotated, while maintaining the same axial position relative to the neck. The protruding elements 112g and the helical groove 111g have translated the rotational movement of the actuator member into an axial movement of the valve member, thereby moving the valve member downward, away from the neck and the actuator member. Hereby, a small opening occurs between the valve member and the neck.

In Fig. 7c, the rotational movement of the actuator member 111 has continued, thereby moving the valve member further downward, to fully expose the openings 112c to the interior of the container. Hereby, an open pathway from the outside, through the actuator member, the valve member and the neck is provided, e.g. enabling an irrigation liquid to be filled into the container.

The neck may be formed by the same material as the rigid top and/or bottom portions of the container, such as a hard plastic material. The actuator member and the valve member may also be made of a hard plastic material, such as polyethylene terephthalate (PET). The gasket may be formed of a softer, more flexible material, such as rubber or a TPE material.

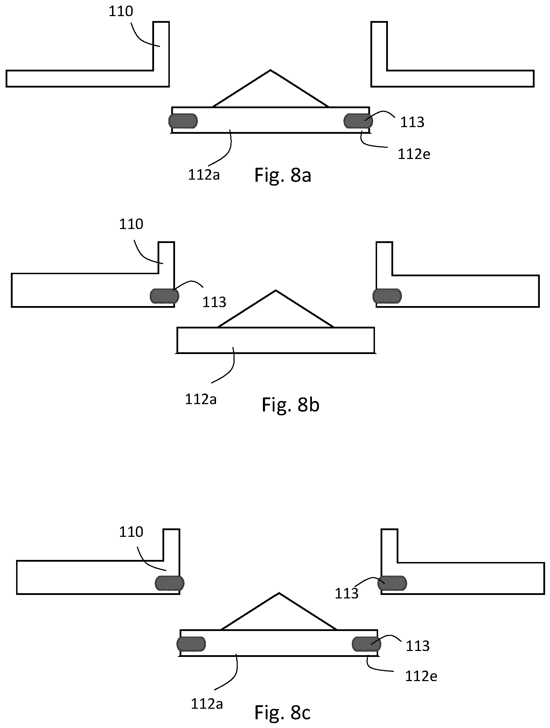

In the above-discussed embodiment, the gasket 113 is arranged in a radially outwardly facing annular groove 112e of the valve bottom 112a, thereby arranged to bear against the inner surface of the neck and form a seal thereto in the closed position. Such an embodiment is schematically illustrated in Fig. 8a.

However, the gasket 113 may alternatively be arranged on, or integrated with, the neck 110, as schematically shown in Fig. 8b. It is also feasible to use two or more gaskets 113. For example, a gasket may be arranged on, or integrated with, both the neck 110 and the valve bottom 112a, as shown schematically in Fig. 8c.

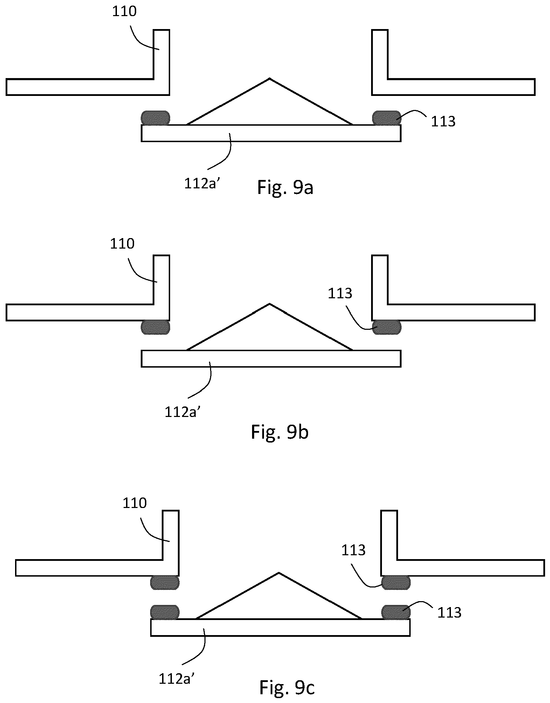

However, the seal may also be arranged between other surfaces, such as an upwardly directed surface of the valve member and a downwardly directed surface of the neck. Such embodiments are shown schematically in Figs. 9a-c. Here, the valve bottom 112a' has an outer diameter greater than the inner diameter of the neck, thereby forming an edge area around the circumference which will abut the neck foot when the valve member is moved upward. This edge area when brought into contact with the foot area of the neck may be sufficient to form the sealed closure. However, alternatively, a gasket may be provided on either the valve bottom 112a', as shown in Fig. 9a, on the foot area of the neck 110, as shown in Fig. 9b, or alternatively on both, as shown in Fig. 9c.

The slanted surface of the valve bottom is preferably rotationally symmetric around a longitudinal axis of the valve member and with the part of the surface being closest to the longitudinal axis being closest to the actuator member. Thus, the central part of the valve bottom may form the highest point of the surface, or alternatively be part of an area of the same height, forming a raised plateau. In the embodiment discussed in the foregoing, the slanted surface is arranged as a conical surface, and in particular in the shape of a concave cone.

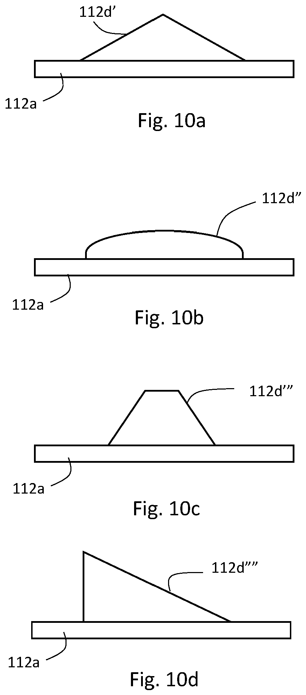

However, other conical surfaces are also feasible, such as a regular, straight cone 112d', as illustrated in Fig. 10a, or a convex cone 112d", as shown schematically in Fig. 10b. Contrary to the concave cone, the convex cone has a less steep, lower inclination at the center, and a steeper, higher inclination at the peripheral edge, and a gradual transition between the low inclination and the high inclination between the center and the periphery.

The slanted surface may have a frustoconical shape 112d'", as shown schematically in Fig. 10c. In such realizations, a smaller or bigger raised plateau is formed in the center of the cone. The rest of the cone may, as before, be straight, convex, or concave.

It is also feasible to have a slanted surface 112d"" which is not rotationally symmetric, but instead slants from one side of the valve member towards another, as shown schematically in Fig. 10d. Also in such realizations, the inclination may be constant, as in the illustrative example, but may also vary, e.g. to form a steeper slope at the most raised parts and a lower inclination at less raised parts.

The actuator member may have a generally circular cross-section, as in the embodiment illustrated in Fig. 4. However, alternatively the actuator member may be arranged to facilitate gripping and maneuvering. For example, as in the above-discussed embodiment, the actuator member may comprise two radially projecting handles 111f extending in opposite directions, thereby forming an oval with pointed ends, such as an almond shape, when seen from above. Such a shape is illustrated e.g. in Fig. 11f.

However, many other shapes are also feasible, such as rectangular, triangular, star shaped, etc. For example, the actuator member may have the shape of a rhombus or rectangle, such as shown in Fig. 11a, when seen from above, where the corners with acute angles form the handles 111f. Alternatively, the actuator member may when seen from above have the shape of a star, with three, four or more arms 111f'. Such a shape is illustrated schematically in Fig. 11b.

Alternatively, as shown in Fig. 11c, the radially projecting handles 111f''' may have a relatively constant cross-sectional shape over their length. It is also feasible to have only one radially projecting handle 111f‴, as in the illustrative example of Fig. 11d.

Further, an actuator member without any projecting handle is also feasible. To increase friction and facilitate gripping and handling, the actuator member may in such realizations have corrugations or the like arranged along the peripheral end surface, as illustrated schematically in Fig. 11e.

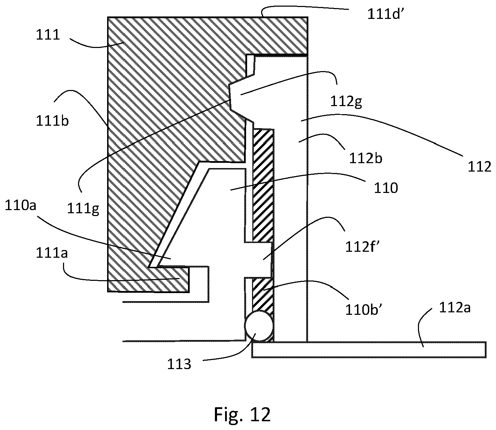

The interaction and engagement between the valve member, neck and actuator member may also be realized in other ways. One such alternative embodiment is illustrated schematically in Fig. 12. Here, the actuator member 111 only comprises an outer tube portion 111b, and an annular flange 111d' extending inwards over the neck opening, but no inner tube portion. The threaded connection between the valve member 112 and the actuator member 111 is here realized as a helical groove 111g arranged in an inwardly facing surface of the outer tube portion 11 1b of the actuator member, and an outwardly protruding thread or pin 112g on an outwardly facing surface of the valve sleeve 112b. The threaded connection translates a rotational movement of the actuator member 111 to an axial movement for the valve member 112. As in previous examples, a longitudinally extending guide groove and a corresponding guide element may be used to hinder or limit rotational movement for the valve member. In this embodiment, the longitudinally extending guide groove 110b' is arranged in an outwardly facing surface of the valve sleeve 112b, whereas the guide element 112f is provided in an inwardly facing surface of the neck 110.

The person skilled in the art realizes that the present invention by no means is limited to the preferred embodiments described above. On the contrary, many modifications and variations are possible within the scope of the appended claims. For example, the engagement between the actuator member and the neck, as well as between the actuator and the valve member, may be provided in other ways. Further, the shape of the actuator member may be varied, as well as where and how to provide the sealed closure between the valve member and the neck. The closure arrangement could also be used for other purposes than filling the container, and the container could be used for other purposes than rectal or transanal irrigation, such as other forms of irrigation, or for other medical procedures. Such and other obvious modifications must be considered to be within the scope of the present invention, as it is defined by the appended claims. It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting to the claim. The word "comprising" does not exclude the presence of other elements or steps than those listed in the claim. The word "a" or "an" preceding an element does not exclude the presence of a plurality of such elements. Further, a single unit may perform the functions of several means recited in the claims.

## Claims

1. A container for medical use forming a closed compartment for accommodating a liquid, comprising:
a neck extending out from a body of the container and defining an opening at a free end of the neck;
an actuator member connected to the neck and arranged to allow rotation of the actuator member relative to the neck in a circumferential direction of the neck but hinder movement of the actuator member relative to the neck in an axial direction of the neck; and
a valve member having a closed valve bottom and a valve sleeve, wherein the valve bottom is connected to the valve sleeve, with an opening formed in a side wall of the valve sleeve in the vicinity of the valve bottom, wherein the valve sleeve is connected to the actuator member, and arranged to interact with the actuator member to be moved in an axial direction relative to the neck and the actuator member upon rotation of the actuator member;
wherein the valve member is axially displaceable between a closed position, in which the valve bottom is arranged relatively closer to the actuator device and a seal is formed between the valve bottom and the neck, and an open position, in which the valve bottom is arranged relatively farther from the actuator device, and wherein the valve bottom forms a slanted surface.

2. The container of claim 1, wherein the neck comprises a first engagement structure extending circumferentially in a closed circular path around an external surface of the neck, and wherein the actuator member comprises a second engagement structure extending circumferentially in a closed circular path around an internal surface of the actuator, the first and second engagement structures being arranged to allow rotation of the actuator member relative to the neck in a circumferential direction of the neck but hinder movement of the actuator member relative to the neck in an axial direction of the neck.

3. The container of claim 1 or 2, wherein the actuator member comprises an outer tube portion and an inner tube portion, the outer and inner tube portions being connected by an annular surface extending between a top end of the outer tube portion and a top end of the inner tube portion, wherein the actuator member is arranged to receive the neck between the outer tube portion and the inner tube portion.

4. The container of claims 2 and 3, wherein said second engagement structure extends circumferentially in a closed circular path around an internal surface of the outer tube portion.

5. The container of claim 3 or 4, wherein the valve sleeve is arranged positioned between the neck and the inner tube portion of the actuator member.

6. The container of any one of the preceding claims, wherein the slanted surface of the valve bottom is rotationally symmetric around a longitudinal axis of the valve member and with the part of the surface being closest to the longitudinal axis being closest to the actuator member.

7. The container of any one of the preceding claims, wherein the slanted surface of the valve bottom forms a conical surface, and preferably a concave cone.

8. The container of any one of the preceding claims, wherein the seal is formed between an outer surface of the valve bottom and an inner surface of the neck, both said outer surface and inner surface being orthogonal to the axial direction of the neck and valve member.

9. The container of any one of the preceding claims, further comprising a gasket, arranged on at least one of the valve bottom and the neck, wherein the gasket is arranged to be positioned between the valve bottom and the neck when the valve member is in the closed position.

10. The container of claim 9, wherein the gasket is arranged on the valve bottom and arranged to protrude radially out past a perimeter of the valve bottom.

11. The container of any one of the preceding claims, wherein the actuator member comprises a radially projecting handle.

12. The container of any one of the preceding claims, wherein the valve member is arranged to move between the closed position and the open position upon less than 180 degrees rotation of the actuator member, and preferably less than 90 degrees, and most preferably less than 60 degrees.

13. The container of any one of the preceding claims, wherein the container comprises a side wall member formed by a flexible material having a first and second open end, a rigid bottom portion sealingly arranged at said first open end of said side wall member and a rigid top portion sealingly arranged at said second open end of said side wall member, wherein the rigid top portion includes said neck.

14. The container of claim 13, wherein the side wall member is flexible such that said container is reversibly foldable and unfoldable, thereby being arrangeable in a compact state, in which the rigid top and bottom portions are arranged relatively closer to each other, and in an expanded state, in which the rigid top and bottom portions are arranged relatively further apart, respectively.

15. The container of claim 13 or 14, wherein the opening in the neck forms a filling opening for the container and wherein a second opening is provided in the rigid bottom portion, said second opening forming a discharge opening.

16. The container according to any one of the preceding claims, wherein the actuator member is operatively connected to the valve member by a threaded connection, formed between an outer surface of the inner tube portion of the actuator member and an inner surface of the valve sleeve of the valve member.

17. The container according to any one of the preceding claims, wherein said container is adapted for use in an irrigation system, and preferably a rectal or transanal irrigation system.

18. An irrigation system, and preferably a rectal or transanal irrigation system, comprising a container in accordance with any one of the preceding claims.

19. A container for medical use forming a closed compartment for accommodating a liquid, comprising:
a neck extending out from a body of the container and defining an opening at a free end of the neck and a first engagement structure extending circumferentially in a closed circular path around an external surface of the neck;
an actuator member having an outer tube portion and an inner tube portion, the outer and inner tube portions being connected by an annular surface extending between a top end of the outer tube portion and a top end of the inner tube portion, wherein the actuator member is arranged to receive the neck between the outer tube portion and the inner tube portion and wherein the outer tube portion has a second engagement structure extending circumferentially in a closed circular path around an internal surface of the outer tube portion, the first and second engagement structures being arranged to allow rotation of the actuator member relative to the neck in a circumferential direction of the neck but hinder movement of the actuator member relative to the neck in an axial direction of the neck; and
a valve member having a closed valve bottom and a valve sleeve, wherein the valve bottom is connected to the valve sleeve, with an opening formed in a side wall of the valve sleeve in the vicinity of the valve bottom, wherein the valve sleeve is arranged to be positioned between the neck and the inner tube portion of the actuator member, and arranged to interact with the actuator member to be moved in an axial direction relative to the neck and the actuator member upon rotation of the actuator member;
wherein the valve member is axially displaceable between a closed position, in which the valve bottom is arranged relatively closer to the actuator device and a seal is formed between the valve bottom and the neck, and an open position, in which the valve bottom is arranged relatively farther from the actuator device, and wherein the valve bottom forms a slanted surface.
